# EUROPEAN PATENT APPLICATION

(11) **EP 4 275 791 A1**
(43) Date of publication of application: **15.11.2023**
(21) Application number: 21917752.4
(22) Date of filing: 27.12.2021
(51) Int. Cl.: B01J 38/04, C07C 47/58, C07C 51/21, C07C 65/21, C07C 49/84, B01J 37/34, B01J 23/72, B01J 23/83, B01J 23/889, B01J 23/94

(54) **LIGNIN-DEGRADING CATALYST, METHOD FOR MANUFACTURING SAME, METHOD FOR DEGRADING LIGNIN, AND METHOD FOR REGENERATING LIGNIN-DEGRADING CATALYST**

(30) Priority: 06.01.2021 JP 2021001016
(71) Applicant: HIROSAKI UNIVERSITY, Hirosaki-shi, Aomori 036-8560 (JP); National University Corporation Hokkaido University, Sapporo-shi, Hokkaido 060-0808 (JP)
(72) Inventor: YOSHIDA, Akihiro, Hirosaki-shi, Aomori 036-8560 (JP); SONOKI, Tomonori, Hirosaki-shi, Aomori 036-8560 (JP); IRWAN, Kurnia, Hirosaki-shi, Aomori 036-8560 (JP); GUAN, Guoqing, Hirosaki-shi, Aomori 036-8560 (JP); ABUDULA, Abuliti, Hirosaki-shi, Aomori 036-8560 (JP); MASUDA, Takao, Sapporo-shi, Hokkaido 060-0808 (JP); YOSHIKAWA, Takuya, Sapporo-shi, Hokkaido 060-0808 (JP)
(74) Representative: Müller-Boré & Partner Patentanwälte PartG mbB
(86) International application number: PCT/JP2021/048664
(87) International publication number: WO 2022/149532

(57) **Abstract**

Provided are a lignin degradation catalyst that exhibits excellent lignin degradability and that is readily separated after degradation reaction, a method for producing the catalyst, and a method for degrading lignin. The lignin degradation catalyst according to the present invention contains a substrate and at least one metal compound immobilized on the substrate, wherein the at least one metal compound contains a copper compound. The method for producing a lignin degradation catalyst according to the present invention includes the step of brining a porous copper substrate into contact with a solution containing an oxidant to obtain a substrate having a copper compound immobilized thereon, or the step of subjecting a porous copper substrate to electro-oxidation to obtain a substrate having a copper compound immobilized thereon.

## Description

### Technical Field

The present invention relates to a lignin degradation catalyst, a method for producing the catalyst, a method for degrading lignin, and a method for regenerating a lignin degradation catalyst.

### Background Art

Lignin is known as a major constituent of wood biomass and is one of the most abundant aromatic carbon resources on earth. To obtain aromatic carbon resources from lignins, it is necessary to perform a reaction that cleaves strong carbon-carbon bonds. Thus, there is demand for a catalyst that allows the reaction to proceed under mild conditions.

Copper compounds are known as one such catalyst and have attracted attention as a useful catalyst for oxidative degradation reaction of lignins. For catalysts for lignin degradation, copper(II) trifluoromethanesulfonate, which is a water-soluble salt, has been proposed (e.g., NPL 1), and Cu/Al₂O₃ in the form of a powder catalyst with copper as the active ingredient has also been reported as showing activity for lignin degradation (e.g., NPL 2).

### Citation List

### Non-patent Literature

NPL 1: Adv. Synth. Catal., 2014, 356, 3563-3574
NPL 2: Green. Chem., 2013, 15, 768-774

### Summary of Invention

### Technical Problem

However, using a water-soluble salt as a catalyst, as in the technique disclosed in NPL 1, results in both the product from degradation reaction and the used catalyst being present together in an aqueous solution after reaction, and this makes it difficult to separate and recover the catalyst. Due to their generally high prices relative to starting materials and generated products and their composition often containing toxic heavy metals, catalysts can complicate processes and increase costs. Using a powder catalyst, as in the technique disclosed in NPL 2, makes it easier for insoluble matter to remain and also makes it easier for solids to form due to re-polymerization of the oxidative degradant of lignin. Thus, in this case as well, it can be difficult to separate the catalyst.

The present invention was made in view of the problems above. An object of the invention is to provide a lignin degradation catalyst that is excellent in degradability of lignin and readily separated after degradation reaction, a method for producing the catalyst, a method for degrading lignin, and a method for regenerating a lignin degradation catalyst.

### Solution to Problem

The present inventors conducted extensive research to achieve the object and found that the object can be achieved by a catalyst including a substrate having at least copper compound formed thereon. Then, they completed the present invention.

Specifically, the present invention includes, for example, the subject matter described in the following items.
Item 1 A lignin degradation catalyst, comprising
   a substrate, and
   at least one metal compound immobilized on the substrate,
   wherein the at least one metal compound is a copper compound.
Item 2 The lignin degradation catalyst according to Item 1, wherein the at least one metal compound contains a copper compound and a compound of metal M with multiple valences.
Item 3 The lignin degradation catalyst according to Item 1, wherein the copper compound is copper hydroxide.
Item 4 The lignin degradation catalyst according to Item 2, wherein the copper compound is copper oxide, and the compound of metal M is an oxide of metal M.
Item 5 The lignin degradation catalyst according to Item 4, wherein the copper compound is copper oxide, and the compound of metal M is cerium oxide.
Item 6 The lignin degradation catalyst according to any one of Items 1 to 5, wherein the substrate is a porous metallic substrate.
Item 7 A method for producing the lignin degradation catalyst of any one of Items 1 to 6, comprising the following step 1A or step 1B:
   step 1A: brining a porous copper substrate into contact with a solution containing an oxidant to obtain a substrate having a copper compound immobilized thereon; and
   step 1B: subjecting a porous copper substrate to electro-oxidation to obtain a substrate having a copper compound immobilized thereon.
Item 8 The method according to Item 7, wherein the oxidant is a persulfate.
Item 9 A method for producing the lignin degradation catalyst of any one of Items 1 to 6, comprising
   step 1C: immersing a substrate in a solution containing a copper source to perform hydrothermal synthesis, thereby obtaining a substrate having a copper compound immobilized thereon.
Item 10 The method according to any one of Items 7 to 9, further comprising
   step 2: brining the substrate having a copper compound immobilized thereon into contact with a solution containing a metal M source, wherein the metal M has multiple valences.
Item 11 A method for degrading lignin, comprising the step of degrading lignin in the presence of the lignin degradation catalyst of any one of Items 1 to 6.
Item 12 The method according to Item 11, wherein the degrading lignin is performed in flow mode.
Item 13 A method for regenerating a lignin degradation catalyst, comprising the step of heating the lignin degradation catalyst of any one of Items 1 to 6.

The lignin degradation catalyst of the present invention exhibits excellent lignin degradability and readily separated after degradation reaction.

### Brief Description of Drawings

Fig. 1(a) shows the results of XRD measurement of the lignin degradation catalyst obtained in Example 1, and Fig. 1(b) shows the results of H₂-TPD measurement (temperature-programmed desorption analysis).
Fig. 2 shows the results of XRD measurement. The upper XRD spectrum shows the results of XRD of the lignin degradation catalyst obtained in Example 1, and the lower XRD spectrum shows the results of XRD of the lignin degradation catalyst obtained in Example 2.
Fig. 3 shows the results of XRD measurement. The upper XRD spectrum shows the results of XRD of the lignin degradation catalyst obtained in Example 1, and the lower XRD spectrum shows the results of XRD of the lignin degradation catalyst obtained in Example 4.
Fig. 4 shows SEM images of the surface of the lignin degradation catalyst obtained in Example 1.
Fig. 5 shows an SEM image of the surface of the lignin degradation catalyst obtained in Example 2.
Fig. 6 shows an SEM image of the surface of the lignin degradation catalyst obtained in Example 4.
Fig. 7 shows a temperature profile in a lignin degradation test.
Fig. 8 shows XRD spectra before and after a test for tolerance for repetition performed by using the catalyst obtained in Example 1.
Fig. 9 shows SEM images before and after the test for tolerance for repetition performed by using the catalyst obtained in Example 1.
Fig. 10 shows XRD spectra before and after a test for tolerance for repetition performed by using the catalyst obtained in Example 2.
Fig. 11 shows SEM images before and after the test for tolerance for repetition performed by using the catalyst obtained in Example 2.
Fig. 12(a) shows an SEM image before a test for tolerance for repetition. Fig. 12(b) shows an SEM image after the sixth round of the test for tolerance for repetition. Fig. 12(c) shows an SEM image of a regenerated catalyst.
Fig. 13 shows the results of a lignin degradation test (flow reactor).

### Description of Embodiments

The following describes embodiments of the present invention in detail. In the present specification, the terms "comprising," "including," and "containing" include the concepts of comprising, containing, consisting essentially of, and consisting of.

### 1. Lignin Degradation Catalyst

The lignin degradation catalyst according to the present invention contains a substrate and at least one metal compound immobilized on the substrate, wherein the at least one metal compound is a copper compound. The lignin degradation catalyst according to the present invention is usable as a catalyst for degrading lignin. The lignin degradation catalyst according to the present invention exhibits excellent lignin degradability and is readily separated after degradation reaction.

The substrate can be of any type, and a wide range of known substrates are usable. In particular, the substrate is preferably a substrate made of a metal (metal substrate). Examples of metal substrates include substrates made of a single metal, such as copper, nickel, titanium, or iron, and substrates made of alloy.

From the viewpoint of ease of producing the lignin degradation catalyst and excellence in lignin degradability, the substrate is preferably a porous substrate, especially preferably a porous metal substrate (i.e., metal foam), more preferably copper foam or nickel foam, and particularly preferably copper foam.

The substrate can be obtained according to, for example, a known production method, or from commercial sources. The shape of the substrate can be selected according to the intended use and required performance. From the viewpoint of ease of separating the catalyst after degradation reaction, the substrate can be in a form other than powder and particles; for example, the substrate can be in the form of sheet, plate, rod, mesh, or disc. The substrate is preferably a plate or disc in shape.

From the viewpoint of ease of handling and ease of separating the catalyst after degradation reaction, the size of the substrate can be set within an appropriate range; for example, the length of the long side of the substrate surface (e.g., the length of one side in the case of a square) is preferably 0.5 cm or more, and more preferably 1 cm or more. The thickness of the substrate is preferably 0.1 cm or more, and more preferably 0.2 cm or more.

In the present invention, a plurality of substrates may be used. A plurality of substrates may be stacked (laminated) one above another for use. In particular, when lignin degradation treatment is performed in flow mode (continuous mode), as described below, it is preferable to stack (laminate) multiple substrates one above another and use the laminate.

In the lignin degradation catalyst of the present invention, at least one metal compound is immobilized on the substrate. The at least one metal compound is a copper compound. Specifically, at least a copper compound is immobilized on the substrate. The copper compound exerts catalytic action in lignin degradation.

Examples of copper compounds include copper oxide (Cu₂O or CuO) and copper hydroxide (Cu(OH)₂). Copper oxide as the copper compound is preferably Cu₂O.

The metal compound immobilized on the substrate may contain substances other than a copper compound. Alternatively, the metal compound immobilized on the substrate may be only the copper compound as described above. However, in this case, unavoidably contained metal compounds other than the copper compound are permitted. The copper compound immobilized on the substrate may be, for example, copper oxide (preferably Cu₂O) or copper hydroxide, or both.

If the metal compound immobilized on the substrate contains metal compounds other than a copper compound, examples of such metal compounds include a compound of metal M with multiple valences.

The metal compound immobilized on the substrate may be two or more types of metal compounds. Specifically, a copper compound and a compound of metal M with multiple valences may be immobilized on the substrate.

Metal M (simply "metal M" below) can be any metal with multiple valences. For example, metal M in oxide form is preferably non-water soluble and stable in an alkali aqueous solution (i.e., resistant to dissolving and degrading in alkaline water). Such metal M includes cerium and manganese, with cerium being preferred.

The compound of metal M can be an oxide or hydroxide of metal M. In particular, the compound of metal M is preferably an oxide of metal M, more preferably an oxide of cerium or manganese, and particularly preferably cerium(IV) oxide (CeO₂).

A metal compound containing a compound of metal M immobilized on the substrate improves the catalytic performance and also the durability of the lignin degradation catalyst, thus, making it less likely for catalytic performance to decrease after repeated use. Especially when the compound of metal M is cerium(IV) oxide (CeO₂), the catalytic performance and durability of the lignin degradation catalyst are likely to improve.

In a metal compound containing a compound of metal M immobilized on the substrate, metal M may be present in any amount. For example, the content of metal M relative to the total content of metals in all the metal compounds immobilized on the substrate is preferably 0.1 to 50% by mass, and more preferably 5 to 30% by mass.

Additionally, a metal compound containing a compound of metal M (in particular, an oxide of metal M) immobilized on the substrate allows the lignin degradation catalyst to be stable even in a strong alkaline aqueous solution and thus makes it less likely for the catalyst to deteriorate even in a strong alkaline environment in which lignin degradation reaction takes place, resulting in excellent catalytic action on lignin degradation and excellent durability. When the compound of metal M is cerium(IV) oxide (CeO₂), the lignin degradation catalyst is particularly stable in a strong alkaline environment.

In the lignin degradation catalyst of the present invention, the metal compound is formed so as to coat the surface of the substrate. The metal compound can be of any shape, such as a granular, fibrous, acicular, or wrinkled shape.

In the case of two or more metal compounds immobilized on the substrate, the layers of the metal compounds can be sequentially stacked to form a laminate, or a single-layered structure formed of multiple metal compounds may be formed. From the viewpoint of ease of exercising catalytic action on lignin degradation, it is preferred that a laminate be formed on the substrate.

In the lignin degradation catalyst of the present invention, the surface of the metal compound immobilized on the substrate may be further covered with another layer. Alternatively, no layer may be formed on the surface of the metal compound immobilized on the substrate; in other words, the topmost layer of the lignin degradation catalyst of the present invention can be the at least one metal compound.

In an example of embodiments of the lignin degradation catalyst of the present invention, copper hydroxide (Cu(OH)₂) is immobilized on the substrate (this embodiment being referred to as "embodiment A"). In embodiment A, the substrate is preferably metal foam, more preferably copper foam or nickel foam, and particularly preferably copper foam. In embodiment A, copper hydroxide immobilized on the substrate is preferably formed, for example, in a spike shape. In embodiment A, copper hydroxide (Cu(OH)₂) is preferably located as the topmost layer of the catalyst.

In another example of embodiments of the lignin degradation catalyst of the present invention, the at least one metal compound immobilized on a substrate is a copper compound and a compound of metal M (this embodiment being referred to as "embodiment B"). In embodiment B, the substrate is preferably metal foam, more preferably copper foam or nickel foam, and particularly preferably copper foam. In embodiment B, the copper compound immobilized on a substrate is copper oxide, and the compound of metal M is an oxide of metal M. In particular, the oxide of metal M is preferably cerium oxide (CeO₂) or manganese oxide (MnO₂), and more preferably cerium(IV) oxide. The compound of metal M is preferably formed so as to coat the copper compound, and the compound of metal M is preferably located as the topmost layer of the catalyst.

Due to its further improved catalytic performance (lignin degradation performance) and its likelihood of exhibiting further improved durability, the lignin degradation catalyst according to embodiment B is less likely to decrease the catalytic performance even after repeated use.

Due to the above configuration, the lignin degradation catalyst of the present invention exhibits excellent lignin degradability and is also more easily separated than conventional powdery catalysts after degradation reaction due to the use of a substrate to immobilize a metal compound that has catalytic activity. Additionally, whereas conventional catalysts in powder form are prone to leaching of metals (in particular, copper), the lignin degradation catalyst of the present invention is less susceptible to metal leaching (in particular, copper) because the metal compound is immobilized on a substrate.

The lignin degraded by using the lignin degradation catalyst of the present invention can be of any type. For example, the lignin degradation catalyst of the present invention can be applied to degradation reaction of various known lignins. For example, the lignin degradation catalyst of the present invention can be used in degrading lignins derived from various wood materials, such as cedar powder and cedar chips.

The production method of the lignin degradation catalyst of the present invention is not limited. For example, a wide range of known methods can be used. In particular, it is preferred that the lignin degradation catalyst of the present invention be produced according to the production method including step 1A, step 1B, or step 1C, described below.

### 2. Method for Producing Lignin Degradation Catalyst

The method for producing the lignin degradation catalyst of the present invention includes the following step 1A, step 1B, or step 1C:
step 1A: brining a porous copper substrate into contact with a solution containing an oxidant to obtain a substrate having a copper compound immobilized thereon;
step 1B: subjecting a porous copper substrate to electro-oxidation to obtain a substrate having a copper compound immobilized thereon;
step 1C: immersing a substrate in a solution containing a copper source to perform hydrothermal synthesis, thereby obtaining a substrate having a copper compound immobilized thereon.

### Step 1A

In step 1A, a porous copper substrate (copper foam) is brought into contact with a solution containing an oxidant. The method of contact is not limited; for example, the method can be immersing a porous copper substrate in a solution containing an oxidant.

The oxidant can be of any type. For example, a wide range of known oxidants can be used in step 1A. The oxidant is preferably water soluble; examples include persulfates and peroxides, of which persulfates are preferable, and ammonium persulfate is particularly preferable.

The solution containing an oxidant used in step 1A is preferably an aqueous solution of an oxidant. The concentration of the oxidant in the solution containing the oxidant is not limited and can be, for example, 0.01 to 5M. When ammonium persulfate is used with a copper foam substrate, the concentration of the oxidant in the solution containing the oxidant is preferably 0.01 to 2M, preferably 0.05 to 1M, and more preferably 0.1 to 0.5M.

The pH of the aqueous solution of an oxidant is not limited and may be, for example, 7 or higher, and more preferably 10 or higher. The pH of the aqueous solution of an oxidant can be adjusted, for example, with a known acidic solution or alkali aqueous solution (e.g., an aqueous solution of sodium hydroxide).

The temperature at which and the time (e.g., immersion time) during which a porous copper substrate is in contact with a solution containing an oxidant are not limited. For example, the temperature may be 10 to 50°C, and the time may be 5 to 300 minutes.

In step 1A, a substrate having a copper compound immobilized thereon is obtained by bringing a porous copper substrate into contact with a solution containing an oxidant. Specifically, such a substrate has copper hydroxide (Cu(OH)₂) immobilized on the surface of copper foam. This substrate may be used as a lignin degradation catalyst, or used in step 2, described below.

### Step 1B

In step 1B, a porous copper substrate is subjected to electro-oxidation. The method for electro-oxidation is not limited, and electro-oxidation can be performed in the same manner as known methods. Generally, it is preferred that electro-oxidation be performed under basic conditions due to ease of preventing copper leaching.

In step 1B, a substrate having a copper compound immobilized thereon is obtained by subjecting a porous copper substrate to electro-oxidation. Specifically, such a substrate has copper hydroxide (Cu(OH)₂) immobilized on the surface of copper foam. This substrate may be used as a lignin degradation catalyst or used in step 2, described below.

### Step 1C

In step 1C, a substrate is immersed in a solution containing a copper source to perform hydrothermal synthesis. The substrate for use in step 1C can be of any type, and is preferably the metal substrate as described above, in particular the metal foam described above.

The copper source for use in step 1C is copper itself or a compound containing copper, and preferably a compound containing copper. Examples of compounds containing copper include oxides, hydroxides, chlorides, inorganic acid salts, and organic acid salts of copper. The inorganic acid salt of copper can be at least one selected from the group consisting of nitrates, sulfates, chlorates, perchlorates, carbonates, bicarbonates, phosphates, and hydrogen phosphates. The organic acid salt of copper can be at least one selected from the group consisting of acetates, oxalates, formates, and succinates.

The solution containing a copper source is preferably an aqueous solution. The concentration of the copper source in the solution containing the copper source is also not limited; the same conditions as known conditions may be applied.

In step 1C, hydrothermal synthesis can be performed, for example, by sealing a container accommodating a solution containing a copper source with a substrate immersed in the solution and then subjecting the container to heat treatment. The temperature in the container during the heat treatment is not limited and can be, for example, 100 to 250°C, and preferably 120 to 180°C. The heating time is also not limited and can be determined according to the heating temperature, such as 6 to 24 hours. The pressure in the container during the heat treatment can also be set as appropriate.

In step 1C, a substrate having a copper compound (e.g., Cu(OH)₂) immobilized thereon can be obtained by performing hydrothermal synthesis. For example, the lignin degradation catalyst in embodiment A can be obtained by performing step 1C. This may be used as a lignin degradation catalyst, or used in step 2, described below.

### Step 2

The method for producing the lignin degradation catalyst of the present invention may further include the following step 2, in addition to step 1A, step 1B, or step 1C.

Step 2: brining the substrate having a copper compound immobilized thereon into contact with a solution containing a metal M source.

In step 2, the metal M source is metal M itself or a compound containing metal M. Metal M is as described above; i.e., metal M is a metal with multiple valences. Metal M includes cerium and manganese, of which metal M is preferably cerium.

The metal M source is preferably a compound containing metal M. Examples of compounds containing metal M include oxides, hydroxides, chlorides, inorganic acid salts, and organic acid salts of metal M. The inorganic acid salt of metal M can be at least one selected from the group consisting of nitrates, sulfates, chlorates, perchlorates, carbonates, hydrogencarbonates, phosphates, and hydrogen phosphates. The organic acid salt of metal M can be at least one selected from the group consisting of acetates, oxalates, formates, and succinates.

In particular, the metal M source is preferably an inorganic acid salt of metal M, and more preferably a nitrate.

The solution containing a metal M source is preferably an aqueous solution. The concentration of the metal M source in a solution containing the metal M source is not limited; for example, the concentration of the metal M source is preferably 0.001 to 1M, preferably 0.005 to 0.5M, more preferably 0.01 to 0.3M, and particularly preferably 0.02 to 0.2M.

The pH of the solution containing a metal M source is not limited; for example, the pH of the solution containing a metal M source is preferably 1 or higher, and more preferably 3 to 14. The pH of the aqueous solution of an oxidant can be adjusted, for example, with an alkali aqueous solution, such as an aqueous solution of sodium hydroxide.

In step 2, the substrate having a copper compound immobilized thereon obtained in step 1A, step 1B, or step 1C is brought into contact with the solution containing a metal M source. The method of contact is not limited; for example, the method can be immersing a substrate having a copper compound immobilized thereon in a solution containing a metal M source.

In step 2, The temperature at which and the time (e.g., immersion time) during which a substrate having a copper compound immobilized thereon is brought into contact with the solution containing a metal M source are not limited. For example, the temperature can be 0 to 150°C, and the time can 5 to 180 minutes. The temperature can be adjusted, for example, by heating the solution containing a metal M source according to an appropriate method. The solution containing a metal M source may be exposed to ultrasonic waves with the substrate having a copper compound immobilized thereon immersed in the solution. If the temperature is adjusted by heating the solution, the solution may be exposed to ultrasonic waves during heating.

In step 2, a lignin degradation catalyst having a structure in which a copper compound is coated with a compound of metal M is obtained by bringing a substrate having the copper compound immobilized thereon into contact with the solution containing a metal M source. This contact causes a redox reaction between copper and metal M; for example, copper hydroxide transforms into copper oxide (e.g., Cu₂O), and metal M increases in valence (e.g., Ce³⁺ changes to Ce⁴⁺). In step 2, for example, the lignin degradation catalyst in embodiment B can be obtained.

Conventional catalysts immobilized on a carrier are produced through, for example, a two-step process in which an active ingredient in particulate form is first prepared and then dispersed and immobilized on a carrier. In contrast, the production method of the present invention enables production of a lignin degradation catalyst through a simplified process.

### 3. Method for Degrading Lignin

In the method for degrading lignin of the present invention, the lignin degradation catalyst of the present invention described above is used in degrading lignin. The method for degrading lignin using the lignin degradation catalyst of the present invention may include, for example, the step of degrading lignin in the presence of the lignin degradation catalyst. This step is referred to as the "oxidative degradation step" below. The lignin to be decomposed includes lignin alone, lignin derivatives, and raw materials, such as wood and grass plants, containing lignins. When lignins in raw materials, such as wood or grass plants, are the target for degradation, these materials may be formed into a dispersion or an eluate.

In the oxidative degradation step, for example, the solution containing lignin in which the lignin degradation catalyst of the present invention has been immersed may be subjected to heat treatment in an oxygen atmosphere in an autoclave. The heat treatment enables degradation treatment of lignin in the solution. Thus, the lignin degradant formed by performing lignin degradation treatment is contained in the solution.

In the oxidative degradation step, the temperature for the heat treatment is not limited, and the same conditions as those for known methods may be applied. For example, the temperature for the heat treatment is 100 to 250°C, and preferably 150 to 200°C. The heat treatment time is also not limited, and can be set according to the temperature. The solution containing lignin is also not limited. For example, the solution may be an alkali solution, specifically an aqueous solution of sodium hydroxide.

In the method for degrading lignin of the present invention, lignin degradation treatment can be performed in batch mode and can also be performed in flow mode (continuous mode). Specifically, the oxidative degradation step can be performed in batch or flow mode (continuous mode). In both the batch mode and the flow mode, the type of the reactor for use is not limited; for example, a wide range of known reactors are usable.

In the oxidative degradation step, if lignin degradation treatment is performed in flow mode (continuous mode), for example, a catalyst bed made by stacking multiple layers of the lignin degradation catalyst may be packed in a tubular container to perform degradative treatment. If lignin degradation treatment is performed in flow mode (continuous mode), the apparatus for use can be selected, for example, from a wide range of known apparatuses. In flow mode (continuous mode), lignin (the target for degradation) to be fed to the catalyst bed can be, for example, in a solution form (e.g., alkali). Oxygen gas may also optionally be flown into the catalyst bed.

Degrading lignin by using the lignin degradation catalyst of the present invention provides a lignin degradant at a high degradation yield. The lignin degradant can be identified and quantified according to the following methods.

First, after the oxidative degradation step is completed, the lignin degradation catalyst is removed from the solution containing a lignin degradant. Water is then added to the solution to dilute it 3-fold or 4-fold, thereby preparing a diluted solution. A portion of the diluted solution is taken out and adjusted to a pH of 3 or lower with an appropriate acid, followed by extracting the degradant of lignin with ethyl acetate three times, thereby obtaining an ethyl acetate extract. Ethyl acetate is distilled off from the ethyl acetate extract, and anhydrous pyridine is added to the obtained solid to prepare a solution. BSTFA (N,O-bistrifluoroacetamide) is added to the solution, and the mixture is heated at 60°C for 30 minutes, thereby obtaining a lignin degradant as a trimethylsilylated product. Thereafter, the trimethylsilylated product as a sample is analyzed by GC-FID and GC-MS to identify and quantify the lignin degradant. Alternatively, the solution adjusted to a pH of 3 or lower with an appropriate acid may be analyzed by HPLC, which can identify and quantify a degradant such as vanillin, vanillic acid, apocynin, syringaldehyde, and syringic acid.

Without wishing to be interpreted in a restrictive manner, the lignin degradant includes, for example, syringic acid, vanillic acid, syringaldehyde, hydroxybenzoic acid, apocynin, vanillin, guaiacol, oxalic acid, glycolic acid, and lactic acid.

### 4. Method for Regenerating Lignin Degradation Catalyst

The lignin degradation catalyst of the present invention can be regenerated according to an appropriate method. An example of such regeneration methods is a method of subjecting the lignin degradation catalyst of the present invention to heat treatment.

The method of subjecting the lignin degradation catalyst to heat treatment is not limited. An example is the method of heating the lignin degradation catalyst at a temperature within the range of 200 to 700°C. The heat treatment time can be set according to the heat treatment temperature, such as 0.5 to 12 hours.

The method of heat treatment is also not limited, and heat treatment can be performed, for example, by using an appropriate heating means, such as a furnace. The atmosphere of the heat treatment is also not limited; for example, heat treatment can be performed in the presence of air, oxygen, or inert gas, with the presence of air or oxygen being preferred.

For example, after the lignin degradation catalyst of the present invention is repeatedly used, the catalytic activity of the lignin degradation catalyst can be recovered by subjecting the catalyst to regeneration treatment as described above. Specifically, the method for regenerating the lignin degradation catalyst of the present invention can efficiently restore catalytic activity and further extend the life of the catalyst.

### Examples

The following describes the present invention in more detail with reference to Examples. However, the present invention is not limited to the embodiments of these Examples.

### Example 1

A porous copper substrate obtained from a commercial source (Xiamen TOB New Energy Technology Co., Ltd.) was cut to an appropriate size to prepare copper foam (1.7 cm × 5 cm, 0.5 g), which was then washed with acetone and distilled water. The copper foam was immersed in a mixture of 100 mL of a 0.12M aqueous solution of ammonium persulfate (oxidant) and 100 mL of a 3M aqueous solution of sodium hydroxide at 30°C for 1 hour. Subsequently, the copper foam was taken out and washed sequentially with distilled water and ethanol, thereby obtaining a lignin degradation catalyst: Cu(OH)₂/CF.

### Example 2

The substrate having copper(II) hydroxide (Cu(OH)₂) immobilized thereon obtained in Example 1 was immersed in a mixture of 50 mL of a 0.03M aqueous solution of Ce(NO₃)₃ and 10 mL of a 0.25M aqueous solution of sodium hydroxide and exposed to ultrasonic waves for 15 minutes. Thereafter, with the mixture kept at 90°C, the substrate was continuously immersed for another 3 hours. Subsequently, the substrate was taken out and washed sequentially with distilled water and ethanol, thereby obtaining a lignin degradation catalyst: Cu₂O@CeO₂/CF.

### Example 3

The same procedure as in Example 2 was performed, except that the 0.03M aqueous solution of Ce(NO₃)₃ was replaced with a 0.03M aqueous solution of Mn(NO₃)₂, thereby obtaining a lignin degradation catalyst: Cu₂O@MnO₂/CF.

### Example 4

Nickel foam was sequentially washed with 1M hydrochloric acid, distilled water, and ethanol and then immersed in a reactor containing 60 mL of an aqueous solution of 1.5 mmol of copper(II) nitrate and 10 mmol of urea, followed by exposing it to ultrasonic waves for 30 minutes. Subsequently, the temperature of the reactor was raised to 170°C and kept for 18 hours. The substrate was then taken out and sequentially washed with distilled water and ethanol, thereby obtaining a lignin degradation catalyst: Cu(OH)₂/NF.

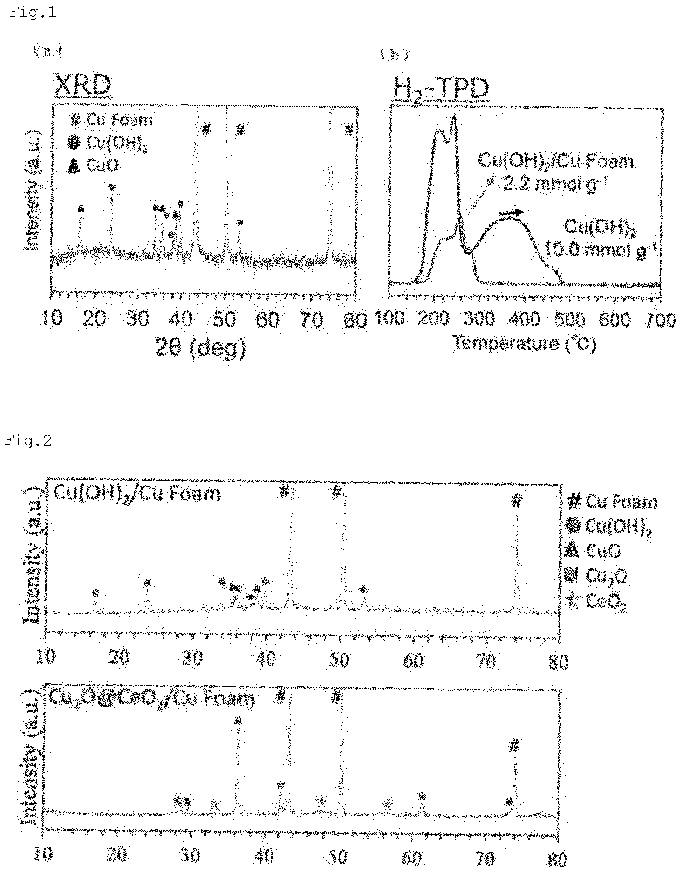

Fig. 1(a) shows the results of XRD measurement of the lignin degradation catalyst obtained in Example 1, and Fig. 1(b) shows the results of H₂-TPR measurement (temperature-programmed desorption analysis). Fig. 1 indicates that the lignin degradation catalyst obtained in Example 1 had Cu(OH)₂ on copper foam.

Fig. 2 shows the results of XRD measurement. The upper XRD spectrum shows the results of XRD of the lignin degradation catalyst obtained in Example 1, and the lower XRD spectrum shows the results of XRD of the lignin degradation catalyst obtained in Example 2. Fig. 2 indicates that the lignin degradation catalyst obtained in Example 2 had copper(I) oxide Cu₂O and cerium(IV) oxide CeO₂ on copper foam.

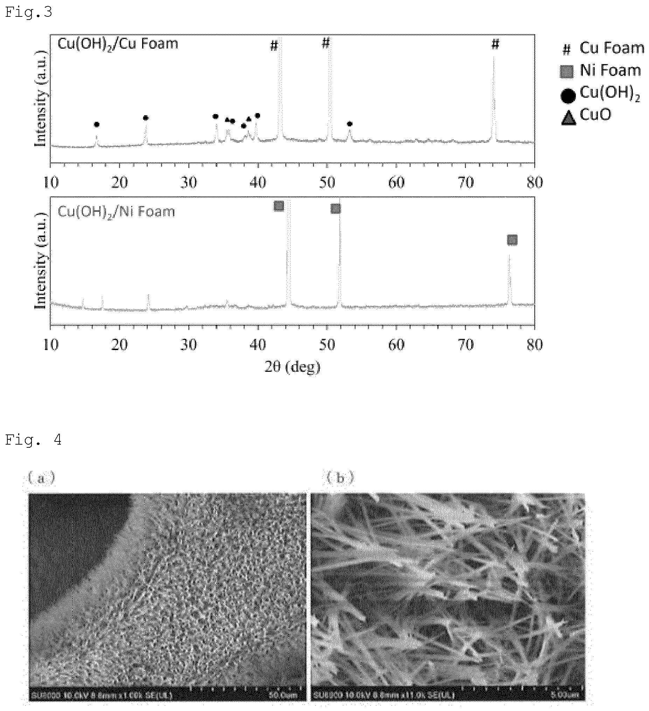

Fig. 3 shows the results of XRD measurement. The upper XRD spectrum shows the results of XRD of the lignin degradation catalyst obtained in Example 1, and the lower XRD spectrum shows the results of XRD of the lignin degradation catalyst obtained in Example 4. Fig. 3 indicates that the lignin degradation catalyst obtained in Example 4 had Cu(OH)₂ on nickel foam.

Figs. 4, 5, and 6 respectively show SEM images of the surface of the lignin degradation catalysts obtained in Examples 1, 2, and 4. (The SEM image in Fig. 4(b) is a magnified view of the SEM image in Fig. 4(a).) Fig. 4 indicates that the surface of the lignin degradation catalyst obtained in Example 1 had a spikey shape; i.e., spikey copper(II) hydroxide was formed.

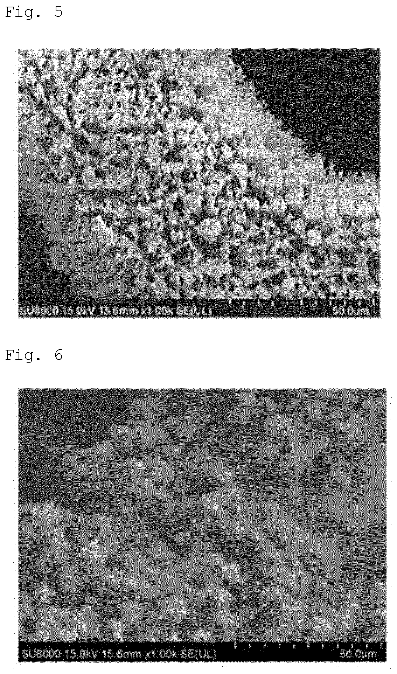

Additionally, EDX measurement performed based on the SEM image of Fig. 5 found that the content of individual elements on the surface was Cu:Ce:O = 1:0.88:2.55 (ratio by mass), and the amount of reducible copper found by H₂-TPR was 1.4 mmolg⁻¹, indicating that cerium(IV) oxide formed in the lignin degradation catalyst obtained in Example 2. Thus, the lignin degradation catalyst obtained in Example 2 had copper(I) oxide Cu₂O and cerium(IV) oxide CeO₂ on copper foam, and the surface was presumably cerium(IV) oxide.

### Lignin Degradation Test 1

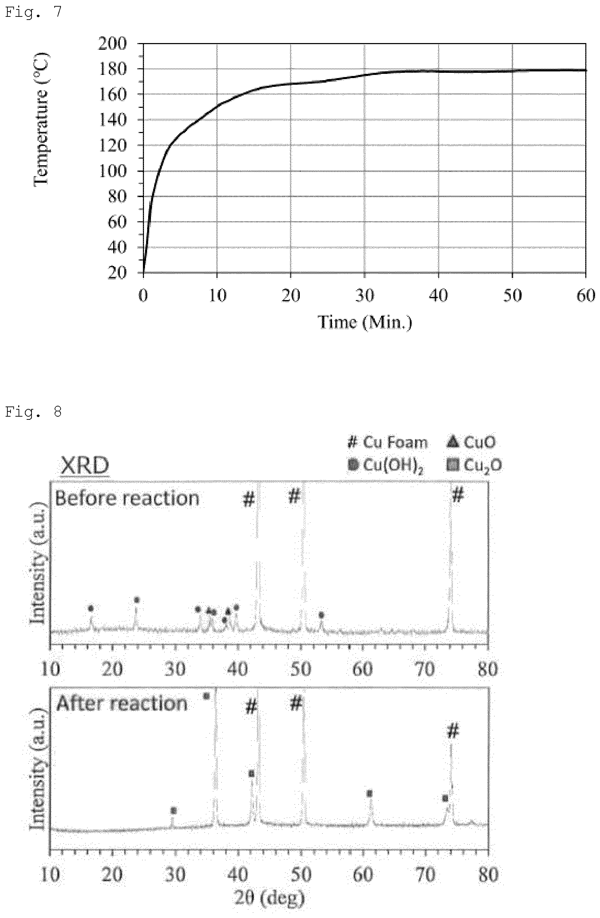

13.5 mL of a 2M aqueous solution of sodium hydroxide containing 0.15 g of commercial sulfite lignin dissolved therein was placed in an autoclave, and the lignin degradation catalysts (weight: 0.5 g) obtained in the Examples were individually immersed in the solution. After air in the autoclave was removed, oxygen was introduced into the autoclave to a pressure of 7 atmosphere, followed by heating with stirring at about 180°C for 25 minutes as in the temperature profile shown in Fig. 7, thereby performing a lignin degradation test.

### Lignin Degradation Test 2

13.5 mL of a 2M aqueous solution of sodium hydroxide containing 0.44 g of cedar powder (the estimated lignin content of which was 0.15 g) dispersed therein was placed in an autoclave, and the lignin degradation catalysts (weight: 0.45 g) obtained in the Examples were individually immersed in the solution. After air in the autoclave was removed, oxygen was introduced into the autoclave to a pressure of 5 atmosphere, followed by heating with stirring at about 180°C for 2 hours as in the temperature profile shown in Fig. 7, thereby performing a lignin degradation test.

### Lignin Degradation Test 3

13.5 mL of a 2M aqueous solution of sodium hydroxide containing 0.44 g of cedar chips (the estimated lignin content of which was 0.15 g) dispersed therein was placed in an autoclave, and the lignin degradation catalysts (weight: 0.45 g) obtained in the Examples were individually immersed in the solution. After air in the autoclave was removed, oxygen was introduced into the autoclave to a pressure of 5 atmosphere, followed by heating with stirring at about 180°C for 2 hours as in the temperature profile shown in Fig. 7, thereby performing a lignin degradation test.

### Lignin Degradation Test 4

The lignin degradation catalysts obtained in the Examples were each cut to a disc (diameter: 10 mm), and 10 discs of each catalyst were placed in a stainless-steel tube (inner diameter: 10 mm) to form a catalyst bed. To the catalyst bed heated to 180°C with a heater, 135 mL of a 2M aqueous solution of sodium hydroxide containing 1.5 g of commercial sulfite lignin dissolved therein was introduced at a flow rate of 0.225 mL per minute by using a feed pump. In addition to the lignin solution, oxygen gas was also introduced into the catalyst bed at a flow rate of 2 mL per minute in standard-state equivalent. A backpressure valve was attached to the outlet of the catalyst bed to keep the pressure inside the catalyst bed at 8 atmosphere by gauge. The outlet solution was periodically collected and analyzed according to the method described in the Examples, and the yield of the main product, vanillin, was calculated.

### Lignin Degradant Identification Method and Degradation Yield Evaluation Method 1

After the lignin degradation test, the lignin degradation catalyst was removed from the solution, and water was added to the solution to dilute it to a total volume of 50 mL, thereby preparing a diluted solution. After 1 mL of the diluted solution was adjusted to a pH of 3 or lower with 30% sulfuric acid, the lignin degradant was washed with 2 mL of ethyl acetate three times, thereby obtaining an ethyl acetate extract. Ethyl acetate was distilled off from the ethyl acetate extract to obtain a solid, and 0.1 mL of anhydrous pyridine was added to the solid to prepare a solution. BSTFA (N,O-bistrifluoroacetamide) was then added to the solution, and the mixture was heated at 60°C for 30 minutes to trimethylsilylate the lignin degradant. Thereafter, the trimethylsilylated product (sample) was analyzed by GC-FID and GC-MS to identify and quantify the lignin degradant.

### Lignin Degradant Identification Method and Degradation Yield Evaluation Method 2

After the lignin degradation test, the lignin degradation catalyst was removed from the solution, and water was added to the solution to dilute it to a total volume of 50 mL, thereby preparing a diluted solution. After 1 mL of the diluted solution was adjusted to a pH of 3 or lower with 30% sulfuric acid, HPLC analysis was performed to identify and quantify vanillin, which was the main product of the lignin degradant, vanillic acid, and apocynin.

In evaluation methods 1 and 2, the yield of the lignin degradant (degradation yield) was calculated from the ratio of the mass of the produced lignin degradant to the mass of the lignin originally contained. The lignin degradant here refers to syringic acid, vanillic acid, syringaldehyde, hydroxybenzoic acid, apocynin, vanillin, guaiacol, oxalic acid, glycolic acid, and lactic acid.

Table 1 shows the results of lignin degradation test 1 for each Example. In lignin degradation test 1, the yield of the lignin degradant (degradation yield) was measured according to evaluation method 1. For reference, Table 1 also shows the results of a lignin degradation test performed without using a catalyst (Reference Example 1), the results of a lignin degradation test using a copper hydroxide powder (Reference Example 2), the results of a lignin degradation test using a copper oxide powder (Reference Example 3), the results of a lignin degradation test using a cerium oxide powder (Reference Example 4), and the results of a lignin degradation test using (untreated) commercial copper foam (Reference Example 5). In Reference Examples 2 and 3, the test was performed in the same manner as in the lignin degradation test above, except that the lignin degradation catalyst obtained in the Examples was replaced with a copper hydroxide powder or copper oxide powder.

Table 1 indicates that the catalysts obtained in the Examples were capable of efficiently degrading lignin and had catalytic performance equivalent to or greater than that of conventional powder catalysts.

**Table 1**

| Example/ Reference Example | Catalyst | Degradation Yield (%) |
|---|---|---|
| Example 1 | Cu(OH)₂/CF | 9.0 |
| Example 2 | Cu₂O@CeO₂/CF | 13.1 |
| Example 3 | Cu₂O@MnO₂/CF | 11.3 |
| Example 4 | Cu(OH)₂/NF | 10.5 |
| Reference Example 1 | No Catalyst | 8.2 |
| Reference Example 2 | Cu(OH)₂ | 12.3 |
| Reference Example 3 | Cu₂O | 12.3 |
| Reference Example 4 | CeO₂ | 9.6 |
| Reference Example 5 | Copper Foam | 7.8 |

**Table 2**

| Example and Catalyst | Tolerance for Repetition | | | | |
|---|---|---|---|---|---|
| | 1 | 2 | 3 | 4 | 5 |
| Example 1 | 100 | 86 | 73 | 57 | No Data |
| Cu(OH)₂/CF | | | | | |
| Example 2 | 100 | 84 | 75 | 69 | 67 |
| Cu₂O@CeO₂/CF | | | | | |

Table 2 shows the evaluation results of tolerance for repetition of the catalysts obtained in Examples 1 and 2. In this evaluation, the yield of the lignin degradant (degradation yield) was measured according to evaluation method 1. The tolerance for repetition of the catalysts was evaluated from the values determined by repeating the lignin degradation test described above up to six times with the catalyst obtained in Example 1 or 2 and calculating the incremental decrease in the yield of the degradant in each round of testing. Specifically, the rate of decrease in the degradation yield of each round of testing relative to the degradation yield in the first lignin degradation test was calculated based on the degradation yield of the first lignin degradation test (100), and the tolerance for repetition of each catalyst was determined.

Table 2 indicates that the catalysts obtained in Examples 1 and 2 both had excellent tolerance for repetition; in particular, the catalyst of Example 2, in which both copper(I) oxide (Cu₂O) and cerium(IV) oxide (CeO₂) formed, had greater tolerance for repetition.

Fig. 8 shows XRD spectra before and after the test for tolerance for repetition performed by using the catalyst obtained in Example 1. The upper XRD spectrum of Fig. 8 shows the results of XRD before the test for tolerance for repetition, and the lower XRD spectrum shows the results of XRD after the fourth round of testing for tolerance for repetition.

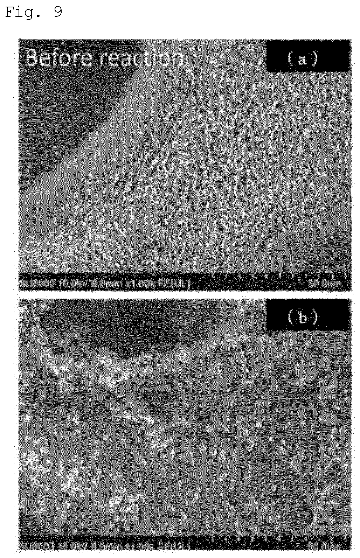

Fig. 9 shows SEM images of the surface of the catalyst obtained in Example 1 before and after the test for tolerance for repetition performed by using the catalyst of Example 1. Fig. 9(a) shows an SEM image before the test for tolerance for repetition, and Fig. 9(b) shows an SEM image after the fourth round of testing for tolerance for repetition.

From Figs. 8 and 9, the catalyst obtained in Example 1 was confirmed to have an ability to tolerate repetition although there were some changes on the surface of the catalyst before and after the test for tolerance for repetition.

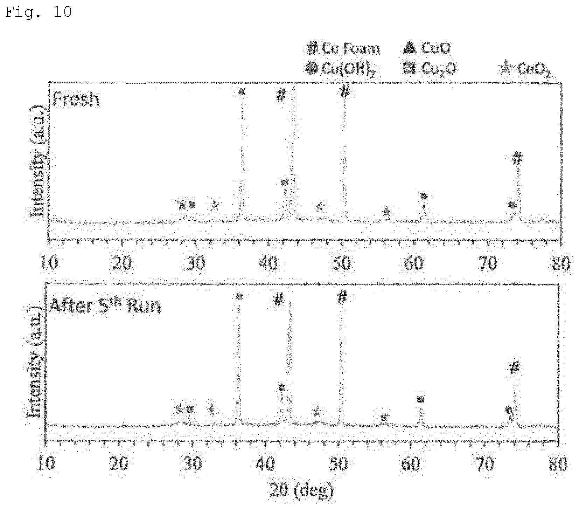

Fig. 10 shows XRD spectra before and after the test for tolerance for repetition performed by using the catalyst obtained in Example 2. The upper XRD spectrum of Fig. 10 shows the results of XRD before the test for tolerance for repetition, and the lower XRD spectrum shows the results of XRD after the fifth round of testing for tolerance for repetition.

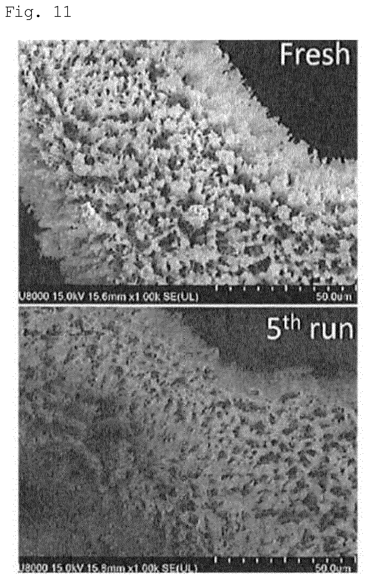

Fig. 11 shows SEM images of the surface of the catalyst obtained in Example 2 before and after the test for tolerance for repetition performed by using the catalyst of Example 2. Fig. 11(a) shows an SEM image before the test for tolerance for repetition, and Fig. 11(b) shows an SEM image after the fifth round of testing for tolerance for repetition.

From Figs. 10 and 11, the catalyst obtained in Example 2 was confirmed to have a greater ability to tolerate repetition than the catalyst of Example 1 because there was no change in either the XRD or SEM images before and after the test for tolerance for repetition.

### Regeneration of Catalyst

In the test for tolerance for repetition described above, the catalyst (the catalyst obtained in Example 2) that underwent the sixth round of testing was subjected to heat treatment at 500°C for 3 hours to regenerate the catalyst. Then, a lignin degradation test was performed in the same manner as above by using the regenerated catalyst. The results showed an increase in degradation yield to 12% in the lignin degradation test using the regenerated catalyst, from the degradation yield of 8.6% in the sixth round of testing performed using the catalyst obtained in Example 2.

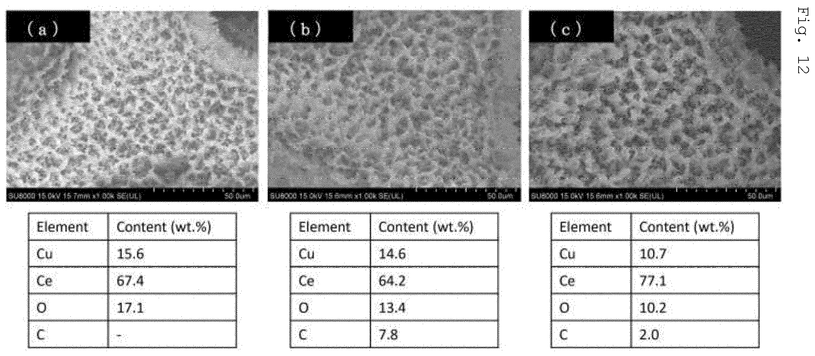

Fig. 12(a) shows an SEM image before the test for tolerance for repetition. Fig. 12(b) shows an SEM image after the sixth round of testing for tolerance for repetition. Fig. 12(c) shows an SEM image of the regenerated catalyst. EDX measurement results based on SEM images are also shown below each SEM image. Fig. 12 indicates that the regenerated catalyst has the same microstructure as that of the catalyst before use. The results above reveal that the regenerated catalyst has improved catalytic activity compared with the catalyst before regeneration.

Table 3 shows the results of lignin degradation test 2 for each Example. In lignin degradation test 2, the yield of the lignin degradant (degradation yield) was measured according to evaluation method 2. For reference, Table 3 also shows the results of a lignin degradation test performed by using a copper hydroxide powder (Reference Example 6). In Reference Example 6, the test was performed in the same manner as in the lignin degradation test above, except that the lignin degradation catalyst obtained in the Examples was replaced with a copper hydroxide powder. The degradation yield shown in Table 3 indicates the total yield of vanillin, vanillic acid, and apocynin.

Table 3 indicates that the catalysts obtained in the Examples were capable of efficiently degrading lignin and had catalytic performance equivalent to that of conventional powder catalysts.

**Table 3**

| Example/ Reference Example | Catalyst | Degradation Yield (%) |
|---|---|---|
| Example 1 | Cu(OH)₂/CF | 15.0 |
| Example 2 | Cu₂O@CeO₂/CF | 12.8 |
| Reference Example 6 | Cu(OH)₂ | 13.1 |

Table 4 shows the results of lignin degradation test 3 for each Example. In lignin degradation test 3, the yield of the lignin degradant (degradation yield) was measured according to evaluation method 2. For reference, Table 4 also shows the results of a lignin degradation test performed by using a copper hydroxide powder (Reference Example 7) and the results of a lignin degradation test performed without using a catalyst (Reference Example 8). In Reference Example 7, the test was performed in the same manner as in the lignin degradation test above, except that the lignin degradation catalyst obtained in the Examples was replaced with a copper hydroxide powder. In Example 1 (using an eluate), shown in Table 4, an eluate was used which was obtained by immersing 0.44 g of the cedar chips used in lignin degradation test 3 in 13.5 mL of a 2M aqueous solution of sodium hydroxide at 180°C for 2 hours. In this elution, the air in the container was replaced with argon to avoid oxidation. The degradation yield shown in Table 4 is the total yield of vanillin, vanillic acid, and apocynin on a carbon atom basis.

Table 4 indicates that the catalysts obtained in the Examples were capable of efficiently degrading lignin and had catalytic performance equivalent to or greater than conventional powder catalysts.

**Table 4**

| Example/Reference Example | Catalyst | Degradation Yield (%) |
|---|---|---|
| Example 1 | Cu(OH)₂/CF | 18.4 |
| Example 1 (using an elute) | Cu(OH)₂/CF | 23.5 |
| Example 2 | Cu₂O@CeO₂/CF | 15.7 |
| Reference Example 7 | Cu(OH)₂ | 18.4 |
| Reference Example 8 | No Catalyst (Ar replacement) | 2.3 |

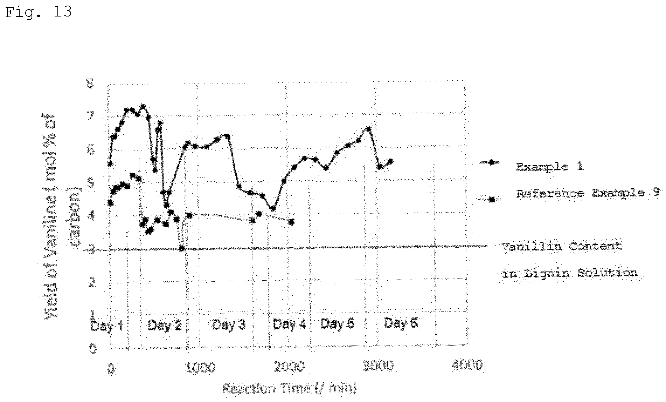

Fig. 13 shows the results of lignin degradation test 4 for each Example. For reference, Fig. 13 also shows the results of a lignin degradation test performed by using a copper hydroxide powder (Reference Example 9). In Reference Example 9, the test was performed in the same manner as in the lignin degradation test above, except that the lignin degradation catalyst obtained in the Examples was replaced with a copper hydroxide powder.

Fig. 13 indicates that the catalysts obtained in the Examples had greater lignin degradation performance than the powder catalyst (Reference Example 9) and could maintain the vanillin yield on a carbon basis of 5.5% even 50 hours after the start of reaction.

## Claims

1. A lignin degradation catalyst, comprising
a substrate, and
at least one metal compound immobilized on the substrate,
wherein the at least one metal compound is a copper compound.

2. The lignin degradation catalyst according to claim 1, wherein the at least one metal compound contains a copper compound and a compound of metal M with multiple valences.

3. The lignin degradation catalyst according to claim 1, wherein the copper compound is copper hydroxide.

4. The lignin degradation catalyst according to claim 2, wherein the copper compound is copper oxide, and the compound of metal M is an oxide of metal M.

5. The lignin degradation catalyst according to claim 4, wherein the copper compound is copper oxide, and the compound of metal M is cerium oxide.

6. The lignin degradation catalyst according to any one of claims 1 to 5, wherein the substrate is a porous metallic substrate.

7. A method for producing the lignin degradation catalyst of any one of claims 1 to 6, comprising the following step 1A or step 1B:
step 1A: a porous copper substrate into contact with a solution containing an oxidant to obtain a substrate having a copper compound immobilized thereon; and
step 1B: subjecting a porous copper substrate to electro-oxidation to obtain a substrate having a copper compound immobilized thereon.

8. The method according to claim 7, wherein the oxidant is a persulfate.

9. A method for producing the lignin degradation catalyst of any one of claims 1 to 6, comprising
step 1C: immersing a substrate in a solution containing a copper source to perform hydrothermal synthesis, thereby obtaining a substrate having a copper compound immobilized thereon.

10. The method according to any one of claims 7 to 9, further comprising
step 2:the substrate having a copper compound immobilized thereon into contact with a solution containing a metal M source, wherein the metal M has multiple valences.

11. A method for degrading lignin, comprising the step of degrading lignin in the presence of the lignin degradation catalyst of any one of claims 1 to 6.

12. The method according to claim 11, wherein the degrading lignin is performed in flow mode.

13. A method for regenerating a lignin degradation catalyst, comprising the step of heating the lignin degradation catalyst of any one of claims 1 to 6.
